# EUROPEAN PATENT APPLICATION

(11) **EP 1 095 623 A1**
(43) Date of publication of application: **02.05.2001**
(21) Application number: 99203577.4
(22) Date of filing: 29.10.1999
(51) Int. Cl.: A61B 17/068, A61B 17/064

(54) **Device for closing a wound or part thereof**

(71) Applicant: Budev Medical B.V., 5271 AT St. Michielsgestel (NL)
(72) Inventor: Jeekel, Johannes, 3062 HM Rotterdam (NL); Paping, Max Gregor, 5271 AT St. Michielsgestel (NL)
(74) Representative: Bruin, Cornelis Willem

(57) **Abstract**

The invention provides a method for closing a wound or part thereof in skin and muscle layer, comprising of bringing together the wound edges in the muscle layer, arranging a number of staples in length direction of the wound at regular chosen mutual distances, and then separately suturing the skin. The invention further relates to a device for closing a wound or part thereof in skin and muscle layer, comprising a handle, a cartridge for a number of staples which is releasable relative to the handle, deforming means for deforming the staples, and operating means operable manually from outside for activating the deforming means.

With the method and device according to the invention a wound in skin and muscle layer, such as for instance in the abdominal wall, can be closed in rapid and simple manner, wherein the incidence of postoperative complications is greatly reduced.

## Description

The present invention relates to a method for closing a wound or part thereof in skin and the muscle layer.

Such a method is for instance known for closing the abdominal wall after abdominal surgery.

A large number of abdominal operations are performed annually. For such an operation the abdomen is opened by making an incision in the skin and muscle layer along the centre line of the abdomen, under the midriff. Here is situated a sheet of tissue (fascia) connecting the muscles. The thickness hereof depends on the patient, but generally amounts to a minimum of 10 mm. The length of the applied incision generally varies between about 5 and 40 cm. After the operation the abdominal wall consisting of the muscle layer and fascia is closed, whereafter the skin is then sutured separately.

After the operation the abdomen size can increase by an average of 30% as a consequence of intra-abdominal pressure increase (during sneezing, laughing and/or pressing). As a result the sutures can cut through the tissue and an acute wound rupture can occur ('burst abdomen').

Another very serious complication which can occur after an abdominal operation is splitting apart of the muscle-fascia layer, resulting in a so-called scar tissue rupture (hernia cicatricalis). Scar tissue rupture after abdominal surgery occurs in about 5-15% of patients. As a consequence of the scar tissue rupture another operation will generally have to be performed, sometimes for cosmetic reasons but usually to treat complications, which can vary from pain to obstruction of the intestine resulting in life-threatening situations. Performing such restorative surgery and the treatment of complications entail considerable cost. Nor moreover are these operations without risk, and in 15-60% of the cases restorative surgery is even ineffective.

Diverse suturing techniques are used to close the abdominal wall, wherein the abdominal wall is sutured with needle and thread in different ways. An attempt is made herein to prevent the occurrence of post-operative complications. Until now however, the known techniques have not produced sufficiently satisfactory results.

The object of the present invention is to provide a method for closing a wound in skin and muscle layer with which the above stated complications can be prevented.

This object is achieved by the invention by providing a method comprising of bringing together the wound edges in the muscle layer, arranging a number of staples in length direction of the wound at regular chosen mutual distances, and then separately suturing the skin.

With the method according to the invention a wound in skin and muscle layer, such as for instance the abdominal wall, can be closed in rapid and simple manner, wherein the incidence of post-operative complications is greatly reduced.

Prior to closing of the wound the wound edges of the wound in the muscle layer can be brought together in any suitable manner. For instance using tweezers or by arranging a number of per se known sutures, whereafter a number of staples are then arranged using the method according to the invention. Once the wound in the muscle layer has been closed, the skin is then sutured separately. Suitable techniques for closing the skin are known to the skilled person.

In a preferred embodiment of the method the staples are plastically deformed such that the legs of the staples are arranged with a radius of curvature in the muscle layer. Arranging of the legs with a radius of curvature ensures that the staples engage firmly in the muscle layer and cannot easily become detached from the tissue when the wound is placed under tension.

Staples are preferably provided with arcuate legs, wherein the staples are bent from the middle point of the body of the staples such that the legs are arranged in the muscle layer with a radius of curvature. Because the legs of the staples are arcuate the legs can be "pressed" with the desired radius of curvature into the muscle layer in simple manner by bending the body of the staples, wherein the least possible damage is inflicted upon the muscle tissue.

The chosen mutual distances between arranged staples preferably lie in the range of 1 to 5 cm. At such a spacing between successive staples a firm closure of the wound is obtained, wherein the chance of complications such as a scar tissue rupture are decreased, even when tension is applied to the wound.

According to the invention at least 5 staples are preferably arranged per 10 centimetres of wound length. A good and reliable closure of the wound is obtained with such a "density" of the staples (number of staples per unit of wound length).

The invention further relates to and provides a device for closing a wound or part thereof in skin and muscle layer, comprising a handle, a cartridge for a number of staples which is releasable relative to the handle, deforming means for deforming the staples, and operating means manually operable from outside for activating the deforming means.

With such a device a wound, such as for instance a surgical wound in the abdominal wall, can be closed in simple manner, wherein the chance of complications is greatly reduced.

The device is positioned for instance by the surgeon at the correct position relative to a wound by means of the handle. The surgeon can then activate the deforming means by means of the operating means operable manually from the outside, whereby the staples are plastically deformed such that they are arranged in the muscle layer with a radius of curvature. Because the cartridge is releasable relative to the handle, the cartridge can be removed in simple manner after use, whereafter the cartridge can be refilled with staples or a new cartridge with staples can be arranged.

In a preferred embodiment of the device the deforming means are arranged on the cartridge. Sterility of the instruments used in an operation is very important, for instance for preventing post-operative infections. Because the deforming means are brought into close contact with the wound during use of the device, they must therefore be thoroughly cleaned and sterilized after use. Arranging of the deforming means on the detachable cartridge enables good cleaning and sterilizing of both the cartridge and the deforming means after use.

The cartridge is preferably a cartridge for once-only use. Using a new sterile cartridge with staples each time before use of the device minimizes the danger of infections.

In a further preferred embodiment of the device the staples comprise arcuate legs and the deforming means are embodied such that they bend the staples from the middle point of the body of the staple such that the legs of the staples are arranged in the muscle layer with a radius of curvature. This ensures that the staples are arranged firmly in the muscle layer and that as little tissue damage as possible occurs therein.

In another advantageous embodiment the device further comprises means for maintaining a uniform chosen spacing between the staples. Maintaining uniform spacing between successive staples decreases the chance of complications such as a scar tissue rupture.

According to the invention the means for maintaining the uniform spacing preferably comprise a mutual connecting wire between the staples. The maximum distance between the staples herein corresponds with the length of the connecting wire between the staples. Because the maximum mutual distance between the staples is fixed, this ensures that the surgeon can maintain a uniform spacing in simple manner. Other suitable means for maintaining a uniform spacing between the staples can however also be applied according to the invention.

The staples preferably comprise a biocompatible material. This prevents the arranged staples causing undesired tissue reactions, such as inflammatory or allergic reactions, in the tissue in which they are arranged. The biocompatible material can also be a resorbent material. Here the staples must however have a working life of at least about a year in order to ensure proper wound healing.

The biocompatible material preferably comprises steel. This material is particularly suitable since it is not only biocompatible but also has the desired strength to form a firm attachment in the muscle layer.

In a preferred embodiment of the device the steel comprises 16-18% chromium, 10-14% nickel, 2-3% molybdenum, a maximum of 2% manganese, 1% silicon and a maximum of 0.03% carbon.

In an advantageous embodiment of the device the width of the staples lies in the range of 20 to 50 mm. This prevents the possibility of the staples detaching easily if the wound swells. In preference the width of the staples is typically 40 mm.

The arcuate legs of the staples are curved in a radius of curvature such that the depth of the staples in the wound lies in the range of 10-25 mm. The staples will hereby remain firmly attached in the wound, also when there is tension on the wound, while this also prevents damage to other tissues, such as for instance the intestines.

The invention further relates to and provides a cartridge for use in the above described device.

The cartridge is preferably a cartridge for once-only use, so that a new sterile cartridge with staples can be fixed to the handle each time before use. Once-only use of the cartridge minimizes the danger of for instance infections resulting from non-sterile materials.

The invention further relates to the use of the device for closing a wound or a part thereof in skin and muscle layer, wherein the device is placed relative to the wound in the muscle layer such that a number of staples are arranged successively in the length direction of the wound at a regular chosen mutual distance by activating the drive means by means of the operating means.

The invention will be further elucidated with reference to the annexed figures, in which
figure 1 shows a perspective view of a preferred embodiment of the device according to the invention;
figure 2 shows a perspective view of a preferred embodiment of the staples;
figure 3 is a perspective view of a preferred embodiment of the device according to the invention, wherein the cartridge is a cartridge for once-only use;
figure 4 shows a preferred embodiment of the deforming means of the device; and
figure 5 shows the use of the device according to the invention for closing a wound in skin and muscle layer.

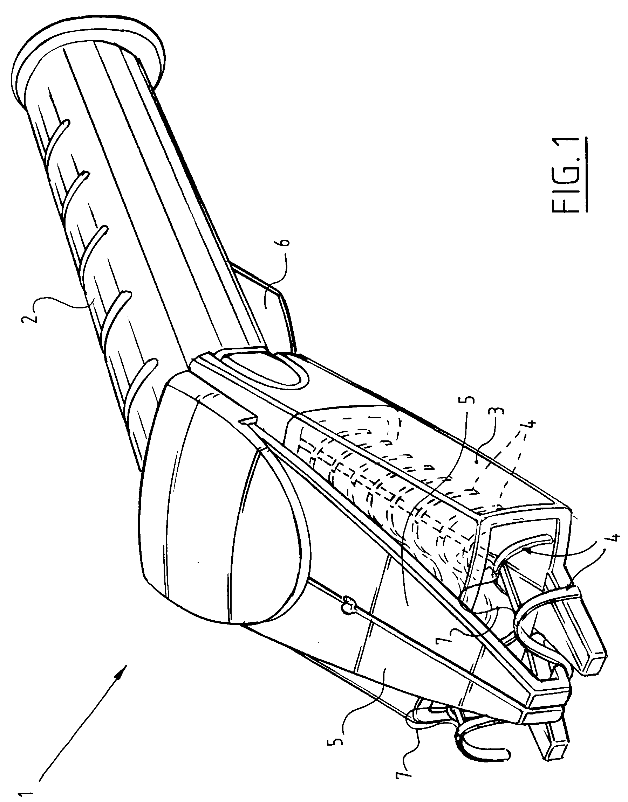

Figure 1 shows a preferred embodiment of the device according to the present invention. Device 1 herein comprises a handle 2, a cartridge 3 for a number of staples 4 which is releasable relative to the handle and deforming means 5 for deforming staples 4. The deforming means can be activated by means of the operating trigger 6 manually operable from outside. Staples 4 are mutually connected by means of a connecting wire 7 to enable maintaining of a uniform distance between the staples arranged in the wound.

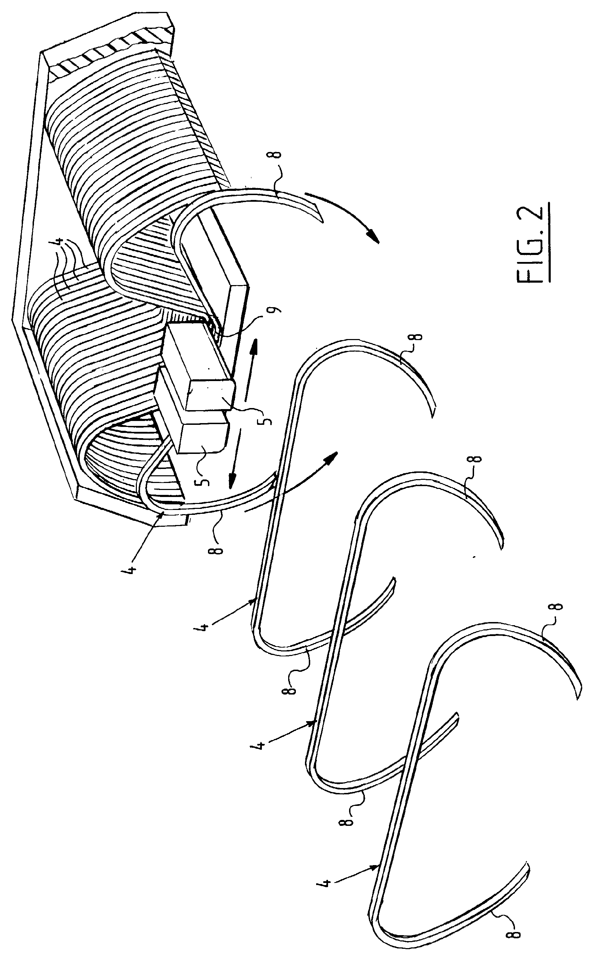

Figure 2 shows the staples in a preferred embodiment of the device according to the invention. Staples 4 herein comprise arcuate legs 8 and the staples are bent from the middle point of the body 9 of the staples by means of deforming arms 5. When the deforming arms are moved apart from the middle of body 9 of the staple the arcuate legs are bent downward and therein pressed into the tissue. Because the legs of the staple are already arcuate, the legs are arranged in the muscle layer with a radius of curvature. This results in a firm and reliable attachment.

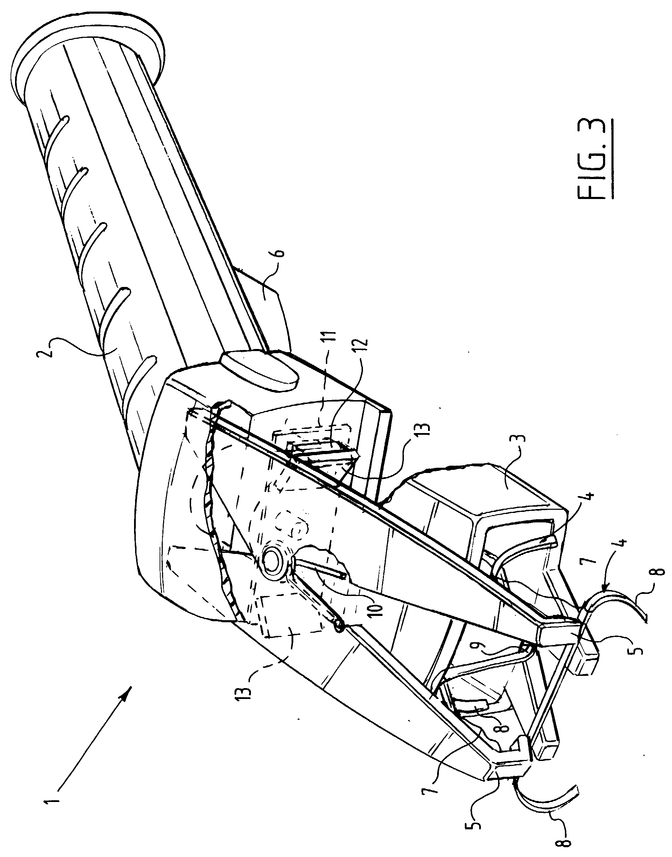

Figure 3 shows a preferred embodiment of the deforming means. The deforming means herein consist of deforming arms 5 mutually connected for pivoting via a torsion spring 10. When the device is activated with operating trigger 6 a pusher member 11 is activated by means of for instance a solenoid, wherein pressing members 12 push against a press-on surface 13 coupled to the deforming arms. Deforming arms 5 are hereby moved apart, wherein the outer ends of the deforming arms bend the arcuate legs 8 from the middle of the body of the staple and therein press them into the tissue. It is noted that figure 2 shows only a preferred embodiment of the device. Other suitable embodiments are also possible according to the present invention.

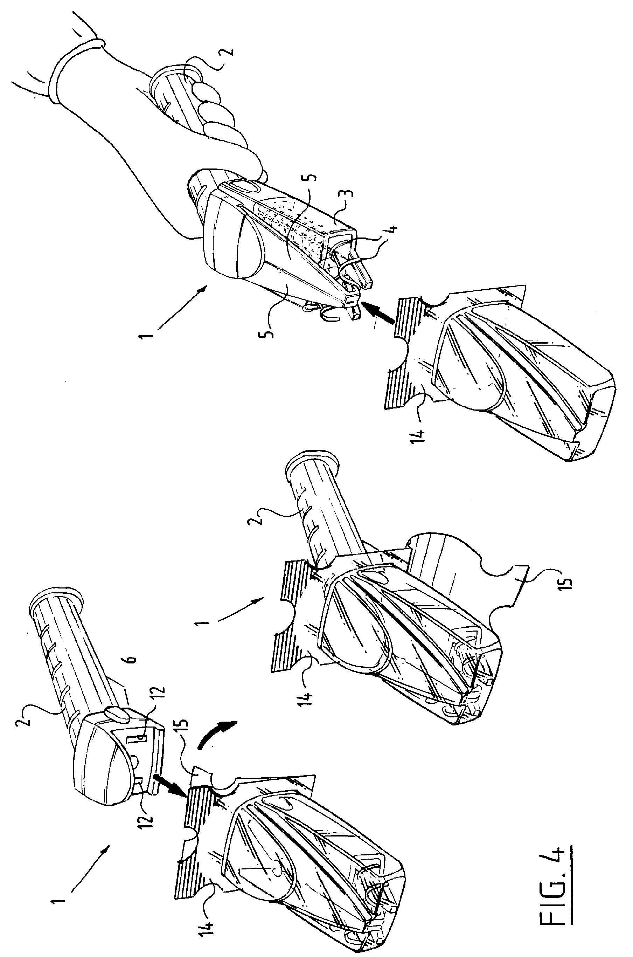

Figure 4 shows how device 1 according to the invention can be used in combination with a cartridge 3 for once-only use. When for instance a surgeon wishes to close the surgical wound after an operation, a cartridge 3 for once-only use in a sterile packaging is coupled to handle 2, without the surgeon herein coming into contact with the cartridge. For this purpose the packaging 14 is opened by removing a protective flap 15, whereafter handle 2 can be coupled to cartridge 3 while the cartridge is still in the packaging. Once the cartridge has been coupled to the handle, device 1 with cartridge 3 can be taken out of the packaging and is ready for immediate use.

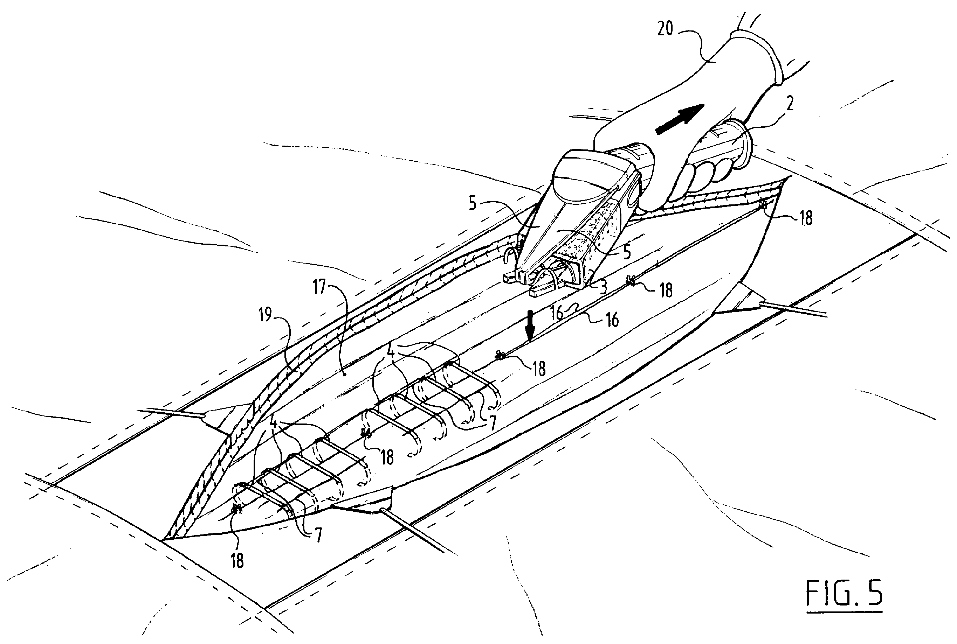

Finally, figure 5 shows the use of the device according to the present invention for closing a wound in skin and muscle layer. Device 1 is positioned by the user 20, such as for instance a surgeon, in lengthwise direction of the wound after wound edges 16 in muscle layer 17 have been brought together using a few sutures 18. A number of staples 4 are then arranged between sutures 18 at mutually regular distances by activating the deforming arms with the operating trigger. After the wound in the muscle layer has been closed the skin 19 is then sutured separately (not shown).

## Claims

1. Method for closing a wound or part thereof in skin and muscle layer, comprising of bringing together the wound edges in the muscle layer, arranging a number of staples in length direction of the wound at regular chosen mutual distances, and then separately suturing the skin.

2. Method as claimed in claim 1, **characterized in that** the staples are plastically deformed such that the legs of the staples are arranged with a radius of curvature in the muscle layer.

3. Method as claimed in claim 2, **characterized in that** staples are provided with arcuate legs and the staples are bent from the middle point of the body of the staples such that the legs are arranged in the muscle layer with a radius of curvature.

4. Method as claimed in claim 1, 2 or 3, **characterized in that** the chosen regular mutual distances lie in the range of 1 to 4 cm.

5. Method as claimed in any of the claims 1-4, characterized in that at least 5 staples are arranged per 10 centimetres of wound length.

6. Device for closing a wound or part thereof in skin and muscle layer, comprising a handle, a cartridge for a number of staples which is releasable relative to the handle, deforming means for deforming the staples, and operating means operable manually from outside for activating the deforming means.

7. Device as claimed in claim 6, **characterized in that** the deforming means are arranged on the cartridge.

8. Device as claimed in claim 6 or 7, **characterized in that** the cartridge is a cartridge for once-only use.

9. Device as claimed in claim 6, 7 or 8, **characterized in that** the staples comprise arcuate legs and the deforming means are embodied such that they bend the staples from the middle point of the body such that the legs of the staples are arranged in the muscle layer with a radius of curvature.

10. Device as claimed in any of the claims 6-9, **characterized in that** the device further comprises means for maintaining a uniform chosen mutual spacing between the staples.

11. Device as claimed in claim 10, **characterized in that** the means for maintaining the uniform spacing comprise a mutual connecting wire between the staples.

12. Device as claimed in any of the claims 6-11, **characterized in that** the staples comprise a biocompatible material.

13. Device as claimed in claim 12, **characterized in that** the biocompatible material comprises steel.

14. Device as claimed in claim 12 or 13, **characterized in that** the steel comprises 16-18% chromium, 10-14% nickel, 2-3% molybdenum, a maximum of 2% manganese, 1% silicon and a maximum of 0.03% carbon.

15. Device as claimed in any of the claims 6-14, **characterized in that** the width of the staples lies in the range of 20 to 50 mm.

16. Device as claimed in claim 15, **characterized in that** the width of the staples is typically 40 mm.

17. Device as claimed in any of the claims 6-16, **characterized in that** the depth of the staples in the wound lies in the range of 10-20 mm.

18. Cartridge for use in a device as claimed in any of the claims 6-17.

19. Cartridge as claimed in claim 18, **characterized in that** the cartridge is a cartridge for once-only use.

20. Use of a device as claimed in any of the claims 6-17 for closing a wound in skin and muscle layer, wherein the device is placed relative to the surgical wound such that a number of staples are arranged successively in the length direction of the wound at a regular chosen mutual distance by activating the deforming means by means of the operating means.
